# EUROPEAN PATENT SPECIFICATION

(11) **EP 2 862 525 B1**
(45) Date of publication and mention of the grant of the patent: **26.10.2016**
(21) Application number: 15150198.8
(22) Date of filing: 12.01.2010
(51) Int. Cl.: A61B 90/11, A61B 34/10, A61B 17/15

(54) **Patella resectioning guide**
Patellaresektionsführung
Guide de resélection de rotule

(30) Priority: 16.01.2009 US 355235
(43) Date of publication of application: 22.04.2015
(62) Divisional of application: 11184387.6
(73) Proprietor: DePuy Products, Inc., Warsaw, IN 46581 (US)
(72) Inventor: Lester, Mark B., Warsaw, IN Indiana 46580 (US); Rhodes, James M., Warsaw, IN Indiana 46582 (US)
(74) Representative: Belcher, Simon James

(56) References cited:
- WO-A1-2008/112996
- US-A1- 2005 234 461

## Description

This invention relates to orthopaedic surgical instruments, especially to a patella resectioning guide.

Joint arthroplasty is a well-known surgical procedure by which a diseased and/or damaged natural joint is replaced by a prosthetic joint. A typical knee prosthesis includes a tibial tray, a femoral component, and a polymer insert or bearing positioned between the tibial tray and the femoral component. In some cases, the knee prosthesis may also include a prosthetic patella component, which is secured to a posterior side of the patient's surgically-prepared patella. To do so, an orthopaedic surgeon may resect the posterior side of the patient's natural patella to secure the prosthetic component thereto. In use, the patella component articulates with the patient's natural or prosthetic femur during extension and flexion of the patient's knee.

To facilitate the replacement of the natural joint with the knee prosthesis, orthopaedic surgeons use a variety of orthopaedic surgical instruments such as, for example, cutting blocks, drill guides, milling guides, and other surgical instruments. Typically, the orthopaedic surgical instruments are generic with respect to the patient such that the same orthopaedic surgical instrument may be used on a number of different patients during similar orthopaedic surgical procedures.

US-A-2005/0234461 discloses instruments for use in resecting a patella which include a cutting block and a resuable exterior piece that includes cutting guides. A compressible foam is secured to an outer surface of the exterior piece.

The present invention provides a patella resectioning guide as defined in claim 1. In some embodiments, the body may have an outer surface opposite the bone-facing surface.

The resectioning guide may further include a medial side corresponding to the medial side of the patient's patella when patella is received in the negative contour of the body. Additionally, the resectioning guide may include a lateral side corresponding to the lateral side of the patient's patella when the patella is received in the negative contour of the body. The cutting slot of the cutting guide may include a first opening on the medial side of the resectioning guide. The first opening may be sized to receive a cutting saw blade. In some embodiments, the cutting slot of the cutting guide may include a second opening on the lateral side of the resectioning guide. The second opening may also be sized to receive a cutting saw blade. Additionally, in some embodiments, the resectioning guide may include an indentation formed on the medial side of the resectioning guide. The indentation may extend from the outer surface of the body to the cutting slot of the cutting guide.

In some embodiments, the body of the resectioning guide may include a sidewall extending upwardly from the bone-facing surface and intersecting with the resectioning plane to prevent the cutting saw blade from extending beyond the body. The sidewall may be positioned on the lateral side of the resectioning guide in some embodiments. Additionally, the cutting guide may be positioned on the medial side of the resectioning guide such that the negative contour of the body may be positioned between the cutting guide and the sidewall.

Additionally, in some embodiments, the outer surface may have an indentation shaped to receive a clamp operable to secure the patella to the body. Additionally (or alternatively), the indentation may be sized to receive a thumb of a surgeon such that the body and patella may be held between the thumb and forefinger of the orthopaedic surgeon.

Embodiments of the invention are described below by way of example with reference to the accompanying drawings, in which:
FIG. 1 is a simplified flow diagram of a method for designing and fabricating a customised patient-specific patella resectioning guide;
FIG. 2 is a side elevation view of an embodiment of a customised patient-specific patella resectioning guide.

Terms representing anatomical references, such as anterior, posterior, medial, lateral, superior, inferior, etcetera, may be used throughout the specification in reference to the orthopaedic implants and surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

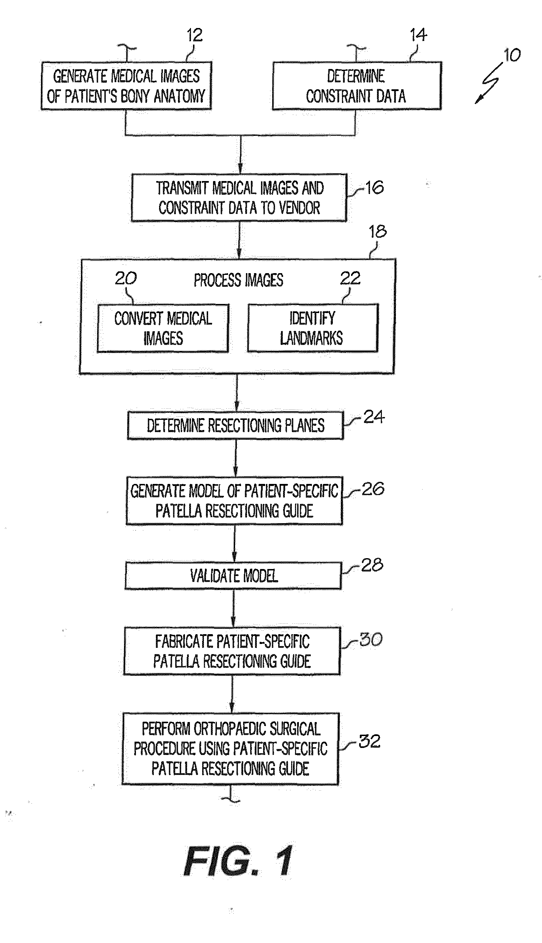

Referring to the drawings, FIG. 1 is a flow diagram with details of a method 10 which can be used to fabricate a customised patient-specific orthopaedic surgical instrument. The term "customised patient-specific orthopaedic surgical instrument" is used in this document to refer to a surgical tool for use by a surgeon in performing an orthopaedic surgical procedure that is intended, and configured, for use on a particular patient. A "customised patient-specific orthopaedic surgical instrument" is distinct from a standard, non-patient specific orthopaedic surgical instrument which is intended for use on a variety of different patients. Additionally, it should be appreciated that the term "customised patient-specific orthopaedic surgical instrument" is distinct from orthopaedic prostheses, whether patient-specific or generic, which are surgically implanted in the body of the patient. Rather, customised patient-specific orthopaedic surgical instruments are used by an orthopaedic surgeon to assist in the implantation of orthopaedic prostheses.

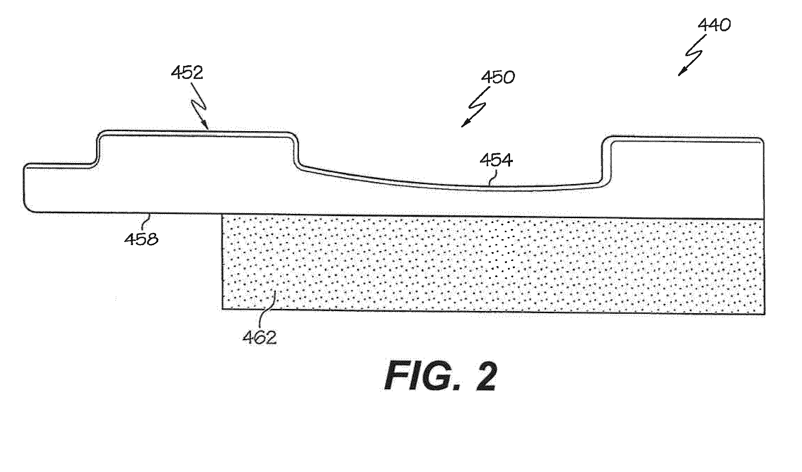

In some embodiments, the customised patient-specific orthopaedic surgical instrument may be customised to the particular patient based on the location at which the instrument is to be coupled to one or more bones of the patient. For example, in some embodiments, the customised patient-specific orthopaedic instrument may be a customised patient-specific patella resectioning guide including one or more bone-contacting or facing surfaces having a negative contour that matches the contour of a portion of the patient's patella, which is discussed in more detail below with reference to FIGS. 2 to 10. As such, the customised patient-specific patella resectioning guide is configured to be coupled to the patient's patella at a unique location and position with respect to the patient's bony anatomy. That is, the negative contours of the bone-contacting surfaces are configured to receive a matching contour surface of the portion of the patient's patella (and, in some cases, femur). As such, the orthopaedic surgeon's guesswork and/or intra-operative decision-making with respect to the placement of the patient-specific patella resectioning guide are reduced. The orthopaedic surgeon may simply couple the customised patient-specific patella resectioning guide to the patient's patella. When so coupled, the customised patient-specific patella resectioning guide defines the thickness of bone the surgeon will resect from the posterior side of the patient's patella.

As shown in FIG. 1, the method 10 includes process steps 12, 14 in which an orthopaedic surgeon performs pre-operative planning of the patella resectioning procedure to be performed on a patient. The process steps 12, 14 may be performed in any order or contemporaneously with each other. In process step 12, a number of medical images of the patient's patella and the surrounding bony anatomy are generated. To do so, the orthopaedic surgeon or other healthcare provider may operate an imaging system to generate the medical images. The medical images may be embodied as any number and type of medical images capable of being used to generate a three-dimensional rendered model of the patient's patella and surrounding bony anatomy. For example, the medical images may be embodied as any number of computed tomography (CT) images, magnetic resonance imaging (MRI) images, or other three-dimensional medical images. Additionally, or alternatively, as discussed in more detail below in regard to process step 18, the medical images may be embodied as a number of X-ray images or other two-dimensional images from which a three-dimensional rendered model of the patient's patella and the surrounding bony anatomy may be generated.

In process step 14, the orthopaedic surgeon may determine any additional pre-operative constraint data. The constraint data may be based on the orthopaedic surgeon's preferences, preferences of the patient, anatomical aspects of the patient, guidelines established by the healthcare facility, or the like. For example, the constraint data may include the orthopaedic surgeon's preference for a particular prosthesis type, the thickness of the bone to resect, the size range of the orthopaedic implant, and/or the like. In some embodiments, the orthopaedic surgeon's preferences are saved as a surgeon's profile, which may be used as a default constraint values for further surgical plans.

In process step 16, the medical images and the constraint data, if any, are transmitted or otherwise provided to an orthopaedic surgical instrument vendor or manufacturer. The medical images and the constraint data may be transmitted to the vendor via electronic means such as a network or the like. After the vendor has received the medical images and the constraint data, the vendor processes the images in step 18. The orthopaedic surgical instrument vendor or manufacturer process the medical images to facilitate the determination of the proper positioning of the prosthetic component, implant sizing, and fabrication of the customised patient-specific patella resectioning guide as discussed in more detail below.

In process step 20, the vendor may convert or otherwise generate three-dimensional images from the medical images. For example, in embodiments wherein the medical images are embodied as a number of two-dimensional images, the vendor may use a suitable computer algorithm to generate one or more three-dimensional images from the number of two-dimensional images. Additionally, in some embodiments, the medical images may be generated based on an established standard such as the Digital Imaging and Communications in Medicine (DICOM) standard. In such embodiments, an edge-detection, thresholding, watershed, or shape-matching algorithm may be used to convert or reconstruct images to a format acceptable in a computer aided design application or other image processing application. Further, in some embodiments, an algorithm may be used to account for tissue such as cartilage not discernable in the generated medical images. In such embodiments, any three-dimensional model of the patient-specific instrument (see, e.g., process step 26 below) may be modified according to such algorithm to increase the fit and function of the instrument.

In process step 22, the vendor may process the medical images, and/or the converted/reconstructed images from process step 20, to determine a number of aspects related to the bony anatomy of the patient such as the anatomical axis of the patient's bones, the mechanical axis of the patient's bone, other axes and various landmarks, and/or other aspects of the patient's bony anatomy. To do so, the vendor may use any suitable algorithm to process the images.

The resectioning plane of the patient's patella is determined in process step 24. The planned resectioning plane is determined based on the type, size, and position of the prosthetic patella component to be used during the orthopaedic procedure, on the process images such as specific landmarks identified in the images, and on the constraint data supplied by the orthopaedic surgeon in previous process steps 14, 16. The type and/or size of the prosthetic patella component may be determined based on the patient's anatomy and the constraint data. For example, the constraint data may dictate the type, make, model, size, or other characteristic of the prosthetic patella component. The selection of the prosthetic patella component may also be modified based on the medical images such that a prosthetic component usable with the bony anatomy of the patient and matching the constraint data or preferences of the orthopaedic surgeon is selected.

In addition to the type and size of the prosthetic patella component, the planned location and position of the prosthetic patella component relative to the patient's bony anatomy is determined. To do so, a digital template of the prosthetic patella component may be overlaid onto one or more of the processed medical images. The vendor may use any suitable algorithm to determine a recommended location and orientation of the prosthetic patella component (i.e., the digital template) with respect to the patient's bone based on the processed medical images (e.g., landmarks of the patient's patella and/or femur defined in the images) and/or the constraint data. Additionally, any one or more other aspects of the patient's bony anatomy may be used to determine the proper positioning of the digital template. In some embodiments, the digital template along with surgical alignment parameters may be presented to the orthopaedic surgeon for approval.

The planned resectioning planes for the patient's patella may then be determined based on the determined size, location, and orientation of the prosthetic patella component. In addition, other aspects of the patient's bony anatomy, as determined in process step 22, may be used to determine or adjust the planned resectioning planes. For example, the determined mechanical axis, landmarks, and/or other determined aspects of the femur and/or patella of the patient may be used to determine the planned resectioning planes.

In process step 26, a model of the customised patient-specific patella resectioning guide is generated. In some embodiments, the model is embodied as a three-dimensional rendering of the customised patient-specific patella resectioning guide. In other embodiments, the model may be embodied as a mock-up or fast prototype of the customised patient-specific patella resectioning guide. The customised patient-specific patella resectioning guide to be modelled and fabricated may be determined based on the patella orthopaedic surgical procedure to be performed, the constraint data, and/or the type of prosthetic patella component to be implanted in the patient.

The particular shape of the customised patient-specific patella resectioning guide is determined based on the planned location of the patella resectioning guide relative to the patient's bony anatomy. The location of the customised patient-specific patella resectioning guide with respect to the patient's bony anatomy is determined based on the type and determined location of the prosthetic patella component to be used during the orthopaedic surgical procedure. That is, the planned location of the customised patient-specific patella resectioning guide relative to the patient's bony anatomy may be selected based on, in part, the planned resectioning planes of the patient's bone(s) as determined in step 24. For example, the location of the patella resectioning guide is selected such that the cutting guide of the patella resectioning guide matches one or more of the planned resectioning planes determined in process step 24. Additionally, the planned location of the patella resectioning guide may be based on the identified landmarks of the patient's patella and femur identified in process step 22.

In some embodiments, the particular shape or configuration of the customised patient-specific patella resectioning guide may be determined based on the planned location of the guide relative to the patient's bony anatomy. That is, the customised patient-specific patella resectioning guide may include a bone-contacting surface having a negative contour that matches a corresponding positive contour of a portion of the patella of the patient. The positive contour of the portion of the patella of the patient may be received in the negative contour of the patella resectioning guide such that the patella is placed in a unique location. When the patella resectioning guide receives the patient's patella, one or more guides (e.g., cutting guide) of the patella resectioning guide may be aligned to the one or more of the resectioning plane(s), as discussed above.

After the model of the customised patient-specific patella resectioning guide has been generated in process step 26, the model is validated in process step 28. The model may be validated by, for example, analysing the rendered model while the three-dimensional model of the patient's patella is received in the resectioning guide model to verify the correlation of the cutting guide and the resectioning plane. Additionally, the model may be validated by transmitting or otherwise providing the model generated in step 26 to the orthopaedic surgeon for review. For example, in embodiments wherein the model is a three-dimensional rendered model, the model along with the three-dimensional images of the patient's relevant bone(s) may be transmitted to the surgeon for review. In embodiments wherein the model is a physical prototype, the model may be shipped to the orthopaedic surgeon for validation.

After the model has been validated in process step 28, the customised patient-specific patella resectioning guide is fabricated in process step 30. The customised patient-specific patella resectioning guide may be fabricated using any suitable fabrication device and method. Additionally, the customised patient-specific patella resectioning guide may be formed from any suitable material such as a metallic material, a plastic material, or combination thereof depending on, as discussed in more detail below. The fabricated customised patient-specific patella resectioning guide is subsequently shipped or otherwise provided to the orthopaedic surgeon. The surgeon performs the orthopaedic surgical procedure in process step 32 using the customised patient-specific patella resectioning guide. As discussed above, because the orthopaedic surgeon does not need to determine the proper location of the patella resectioning guide intra-operatively, which typically requires some amount of estimation on part of the surgeon, the guesswork and/or intra-operative decision-making on part of the orthopaedic surgeon is reduced.

It should also be appreciated that variations in the bony anatomy of the patient may require more than one customised patient-specific patella resectioning guide to be fabricated according to the method described herein. For example, the patient may require the implantation of two prosthetic patella components to replace both natural knees. As such, the surgeon may follow the method 10 of FIG. 1 to fabricate a different customised patient-specific patella resectioning guide for use in replacing each natural knee. Each customised patient-specific patella resectioning guide defines a particular resectioning plane relative to each particular patella that is different due to the variation in the bony anatomy of each knee joint.

FIG. 2 shows a resectioning guide 440 which includes a body 450 and a cutting guide 452 secured to the body 450. The body 450 a bone-facing surface 454 and an outer surface 458 opposite the bone-facing surface. The resectioning guide 440 also includes a compressible foam material 462 secured to the outer surface 458 by a suitable adhesive or other bonding mechanism. The compressible foam material 462 engages with the portion of the distal end of the patient's femur during the resectioning procedure.

In use, the orthopaedic surgeon may place the compressible foam material 462 in contact with the positive contour 49 of the portion of the distal end of the patient's femur. When the patella is secured to the resectioning guide 440, the surgeon may apply pressure to the anterior side of the patient's patella to secure the patella, the resectioning guide 440, and the femur together and perform the resectioning procedure as described above.

## Claims

1. A patella resectioning guide (440) which comprises:
(i) a body (450) including a bone-facing surface (454) having a negative contour configured to receive a portion of a posterior side of a patient's patella that has a corresponding positive contour, and
(ii) a cutting guide (452) including a cutting slot defined therein, in which (i) the cutting slot defines a resectioning plane, and (ii) the cutting guide is positioned such that the resectioning plane extends through the patient's patella when the patella is received in the negative contour of the body,
in which the patella resectioning guide includes a compressible foam material (462) secured to an outer surface (458) of the body, the outer surface being opposite the bone-facing surface of the body,
**characterised in that** the body (450) and the cutting guide (452) are provided as a single monolithic component.

2. The guide (440) as claimed in claim 1, in which the body (450) has an outer surface (458) opposite the bone-facing surface (454), the outer surface having an indentation shaped to receive a clamp configured to secure the patella to the body.

3. The guide (440) as claimed in claim 1, which has:
a medial side corresponding to the medial side of the patient's patella when the patella is received in the negative contour of the body (450), and
a lateral side corresponding to the lateral side of the patient's patella when the patella is received in the negative contour of the body,
in which the cutting slot of the cutting guide (452) includes a first opening on the medial side of the resectioning guide, the first opening being sized to receive a cutting saw blade.

4. The guide (440) as claimed in claim 3, in which the cutting slot of the cutting guide (452) includes a second opening on the lateral side of the resectioning guide, the second opening being sized to receive a cutting saw blade.

5. The guide (440) as claimed in claim 3, which has an indentation (92) formed on the medial side of the resectioning guide, and in which the body (450) has an outer surface (458) opposite the bone-facing surface (454), the indentation extending from the outer surface of the body to the cutting slot of the cutting guide.

6. The guide (440) as claimed in claim 3, in which the body (450) includes a sidewall (158) extending upwardly from the bone-facing surface (454) and intersecting with the resectioning plane to prevent the cutting saw blade from extending beyond the body.

7. The guide (440) as claimed in claim 6, in which:
the sidewall (158) is positioned on the lateral side of the resectioning guide, and
the cutting guide (452) is positioned on the medial side of the resectioning guide such that the negative contour of the body is positioned between the cutting guide and the sidewall.

8. The guide (440) as claimed in claim 1, in which:
the body (450) of the resectioning guide has an outer surface opposite the bone-facing surface, and
the outer surface has an indentation sized to receive a thumb of an orthopaedic surgeon such that the body and patella may be held between the thumb and forefinger of the surgeon.

## Patentansprüche

1. Kniescheibenresektionsführung (440), umfassend:
(i) einen Körper (450), der eine auf einen Knochen gerichtete Oberfläche (454) mit einer negativen Kontur beinhaltet, die zum Aufnehmen eines Abschnitts einer hinteren Seite einer Kniescheibe eines Patienten ausgebildet ist, der eine entsprechende positive Kontur aufweist, und
(ii) eine Schneidführung (452), die einen darin festgelegten Schneidschlitz beinhaltet, wobei (i) der Schneidschlitz eine Resektionsebene festlegt und (ii) die Schneidführung derart angeordnet ist, dass sich die Resektionsebene durch die Kniescheibe des Patienten erstreckt, wenn die Kniescheibe in der negativen Kontur des Körpers aufgenommen ist,
wobei die Kniescheibenresektionsführung ein kompressibles Schaummaterial (462) beinhaltet, das an einer Außenoberfläche (458) des Körpers angebracht ist, wobei die Außenoberfläche der auf einen Knochen gerichteten Oberfläche des Körpers gegenüber liegt,
**dadurch gekennzeichnet, dass** der Körper (450) und die Schneidführung (452) als eine einzelne, einstückige Komponente bereitgestellt sind.

2. Führung (440) nach Anspruch 1, bei welcher der Körper (450) eine Außenoberfläche (458) gegenüber der auf einen Knochen gerichteten Oberfläche (454) aufweist, wobei die Außenoberfläche eine Vertiefung aufweist, die zum Aufnehmen einer Klammer geformt ist, die zum Anbringen der Kniescheibe an dem Körper ausgebildet ist.

3. Führung (440) nach Anspruch 1, die
eine mediale Seite, die der medialen Seite der Kniescheibe des Patienten entspricht, wenn die Kniescheibe in der negativen Kontur des Körpers (450) aufgenommen ist, und
eine laterale Seite aufweist, die der lateralen Seite der Kniescheibe des Patienten entspricht, wenn die Kniescheibe in der negativen Kontur des Körpers aufgenommen ist,
wobei der Schneidschlitz der Schneidführung (452) eine erste Öffnung auf der medialen Seite der Resektionsführung beinhaltet, wobei die erste Öffnung eine Größe zum Aufnehmen eines Schneidsägeblatts aufweist.

4. Führung (440) nach Anspruch 3, bei welcher der Schneidschlitz der Schneidführung (452) eine zweite Öffnung auf der lateralen Seite der Resektionsführung beinhaltet, wobei die zweite Öffnung eine Größe zum Aufnehmen eines Schneidsägeblatts aufweist.

5. Führung (440) nach Anspruch 3, die eine Vertiefung (92) aufweist, die auf der medialen Seite der Resektionsführung ausgebildet ist und wobei der Körper (450) eine Außenoberfläche (458) gegenüber der auf einen Knochen gerichteten Oberfläche (454) aufweist, wobei sich die Vertiefung von der Außenoberfläche des Körpers zu dem Schneidschlitz der Schneidführung erstreckt.

6. Führung (440) nach Anspruch 3, bei welcher der Körper (450) eine Seitenwand (158) beinhaltet, die sich aufwärts von der auf einen Knochen gerichteten Oberfläche (454) erstreckt und sich mit der Resektionsebene schneidet, so dass ein Vorragen des Schneidsägeblatts über den Körper hinaus verhindert wird.

7. Führung (440) nach Anspruch 6, bei der:
die Seitenwand (158) auf der lateralen Seite der Resektionsführung angeordnet ist und
die Schneidführung (452) derart auf der medialen Seite der Resektionsführung angeordnet ist, dass die negative Kontur des Körpers zwischen der Schneidführung und der Seitenwand angeordnet ist.

8. Führung (440) nach Anspruch 1, bei welcher:
der Körper (450) der Resektionsführung eine Außenoberfläche gegenüber der auf einen Knochen gerichteten Oberfläche aufweist, und
die Außenoberfläche eine Vertiefung aufweist, die eine Größe zum Aufnehmen eines Daumens eines orthopädischen Chirurgen aufweist, so dass der Körper und die Kniescheibe zwischen dem Daumen und dem Zeigefinger des Chirurgen gehalten werden können.

## Revendications

1. Ensemble de guide de résection de rotule (440) qui comprend :
(i) un corps (450) comprenant une surface orientée vers l'os (454) ayant un contour négatif configuré pour recevoir une partie d'un côté postérieur de la rotule d'un patient qui a un contour positif correspondant, et
(ii) un guide de coupe (452) qui comprend une fente de coupe définie dans ce dernier, dans lequel (i) la fente de coupe définit un plan de résection, et (ii) le guide de coupe est positionné de sorte que le plan de résection s'étend à travers la rotule du patient lorsque la rotule est reçue dans le contour négatif du corps,
dans lequel le guide de résection de la rotule comprend un matériel en mousse compressible (462) fixé sur une surface externe (458) du corps, la surface externe étant opposée à la surface orientée vers l'os du corps,
**caractérisé en ce que** le corps (450) et le guide de coupe (452) sont formés comme un composant monolithique unique.

2. Guide (440) selon la revendication 1, dans lequel le corps (450) comprend une surface externe (458) opposée à la surface orientée vers l'os (454), la surface externe ayant une indentation formée pour recevoir un clamp configuré pour fixer la rotule au corps.

3. Guide (440) selon la revendication 1, possédant :
un côté médial correspondant au côté médial de la rotule du patient lorsque la rotule est reçue dans le contour négatif du corps (450), et
un côté latéral correspondant au côté latéral de la rotule du patient lorsque la rotule est reçue dans le contour négatif du corps,
dans lequel la fente de coupe du guide de coupe (452) comprend une première ouverture sur le côté médial du guide de résection, la première ouverture étant dimensionnée pour recevoir une lame de scie de coupe.

4. Guide (440) selon la revendication 3, dans lequel la fente de coupe du guide de coupe (452) comprend une deuxième ouverture sur le côté latéral du guide de résection, la deuxième ouverture étant dimensionnée pour recevoir une lame de scie de coupe.

5. Guide (440) selon la revendication 3, qui a une indentation (92) formée sur le côté médial du guide de résection et dans lequel le corps (450) possède une surface externe (458) opposée à la surface orientée vers l'os (454), l'indentation s'étendant à partir de la surface externe du corps jusqu'à la fente de coupe du guide de coupe.

6. Guide (440) selon la revendication 3, dans lequel le corps (450) comprend une paroi latérale (158) s'étendant vers le haut à partir de la surface orientée vers l'os (454) et coupant le plan de résection pour empêcher la lame de scie de coupe de s'étendre au-delà du corps.

7. Guide (440) selon la revendication 6, dans lequel :
la paroi latérale (158) est positionnée sur le côté latéral du guide de résection, et
le guide de coupe (452) est positionné sur le côté médial du guide de résection de sorte que le contour négatif du corps est positionné entre le guide de coupe et la paroi latérale.

8. Guide (440) selon la revendication 1, dans lequel :
le corps (450) du guide de résection a une surface externe opposée à la surface orientée vers l'os, et
la surface externe possède une indentation dimensionnée pour recevoir un pouce d'un chirurgien orthopédique de sorte que le corps et la rotule puissent être maintenus entre le pouce et l'index du chirurgien.
